(19) Europäisches Patentamt
European Patent Office
Office européen des brevets

(11) Veröffentlichungsnummer: **0 071 707**
**B1**

(12) # EUROPÄISCHE PATENTSCHRIFT

(45) Veröffentlichungstag der Patentschrift: **27.12.84**

(51) Int. Cl.³: **C 07 D 309/06**, C 07 D 309/22, C 07 D 307/14, C 07 D 307/16, C 07 D 317/28, C 07 D 317/30, C 07 D 319/06, C 07 D 335/02, C 07 D 339/06, A 01 N 43/02

(21) Anmeldenummer: **82104688.5**

(22) Anmeldetag: **28.05.82**

(54) **Cyclohexandionderivate, Verfahren zu ihrer Herstellung und diese enthaltende Herbizide.**

(30) Priorität: **29.05.81 DE 3121355**

(43) Veröffentlichungstag der Anmeldung:
**16.02.83 Patentblatt 83/7**

(45) Bekanntmachung des Hinweises auf die Patenterteilung:
**27.12.84 Patentblatt 84/52**

(84) Benannte Vertragsstaaten:
**AT BE CH DE FR GB IT LI LU NL SE**

(56) Entgegenhaltungen:
**EP - A - 0 070 370**
**DE - A - 2 439 104**
**DE - A - 2 524 577**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Becker, Rainer, Dr., Sonnenwendstrasse 83, D-6702 Bad Duerkheim (DE)**
Erfinder: **Jahn, Dieter, Dr., Burgunder Weg 8, D-6803 Neckarhausen (DE)**
Erfinder: **Rohr, Wolfgang, Dr., In der Dreispitz 13, D-6706 Wachenheim (DE)**
Erfinder: **Himmele, Walter, Dr., Eichenweg 14, D-6909 Walldorf (DE)**
Erfinder: **Siegel, Hardo, Dr., Hans-Purrmann-Allee 25, D-6720 Speyer (DE)**
Erfinder: **Wuerzer, Bruno, Dr. Dipl.-Landwirt, Ruedigerstrasse 13, D-6701 Otterstadt (DE)**

**EP 0 071 707 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cyclohexan-1,3-dionderivate, Verfahren zur Herstellung dieser Verbindungen sowie Herbizide, welche diese Verbindungen enthalten.

Cyclohexandionderivate mit Thienyl- oder Furylsubstitution in 5-Position mit relativ geringer herbizider Wirkung sind bekannt (DE-AS Nr. 2439104).

Es wurde nun gefunden, dass Verbindungen der allgemeinen Formel I

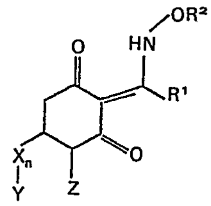

(I)

in der

$R^1$ Alkyl mit 1 bis 4 Kohlenstoffatomen,

$R^2$ Alkyl mit 1 bis Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen,

X geradkettiger oder verzweigter Alkylenrest mit 1 bis 5 Kohlenstoffatomen, gegebenenfalls phenylsubstituiert,

n 0 oder 1,

Y ein nichtaromatischen Heterocyclus mit 5 oder 6 Atomen und keiner oder einer Doppelbindung im heterocyclischen Ring, enthaltend 1 oder 2 Heteroatome aus der Gruppe Schwefel oder Sauerstoff, wobei der Heterocyclus gegebenenfalls substituiert ist durch Alkyl in Form von Methyl, und

Z Wasserstoff oder Methoxycarbonyl

bedeutet sowie die Salze dieser Verbindungen unerwünschte Pflanzen aus der Familie der Gräser sehr gut bekämpfen und gleichzeitig als selektive Herbizide ein hohes Mass an Verträglichkeit für breitblättrige und andere nicht zu der Familie der Gräser zählende Kulturpflanzen besitzen.

$R^1$ bedeutet beispielsweise Propyl, Ethyl, Butyl,

$R^2$ bedeutet beispielsweise Methyl, Ethyl, Propyl, Allyl, 2-Chlorallyl, 3-Chlorallyl,

X bedeutet beispielsweise Methylen, Ethylen, und

Y bedeutet beispielsweise Tetrahydropyranyl, Dihydropyranyl, Methyltetrahydropyranyl, Dioxanyl, Dioxolanyl, Dithiolanyl, Dihydrothiopyranyl.

Die neuen Verbindungen können in verschiedenen tautomeren Formen vorliegen

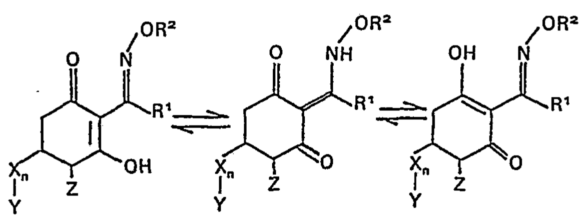

Die vorliegende Erfindung umfasst alle diese Formen.

Zur Herstellung der neuen Verbindungen ist beispielsweise der nachfolgend beschriebene Weg geeignet

a)

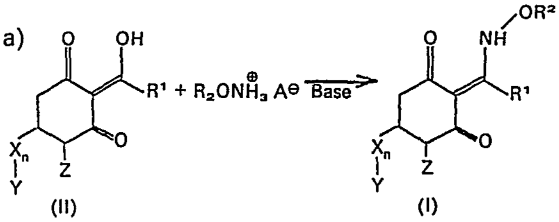


(II) (I)

wobei $R^1$, $R^2$, X, Y, Z, A die oben genannte Bedeutung haben.

Man führt die Reaktion zweckmässig in heterogener Phase in einem inerten Lösungsmittel bei Temperaturen zwischen 0 und 80°C in Gegenwart einer Base durch. Basen sind beispielsweise Carbonate, Hydrogencarbonate, Acetate, Alkoholate, Hydroxide oder Oxide von Alkali- oder Erdalkalimetallen, besonders von Natrium und Kalium sowie Magnesium und Kalium. Daneben können auch organische Basen wie Pyridin oder tertiäre Amine Verwendung finden.

Ein für die Umsetzung besonders geeigneter definierter pH-Bereich reicht von pH 2 bis pH 7, insbesondere von pH 4,5 bis pH 5,5. Die Einstellung des pH-Bereichs für die Umsetzung erfolgt vorteilhaft durch Zusatz von Acetaten, beispielsweise Alkaliacetaten, insbesondere Natrium- oder Kaliumacetat oder ihren Mischungen. Die Alkaliacetate werden beispielsweise angewendet in Mengen von 0,5 bis 2 mol, bezogen auf die Ammoniumverbindung.

Als Lösungsmittel sind geeignet beispielsweise Methanol, Ethanol, Isopropanol, Benzol, Tetrahydrofuran, Chloroform, Acetonitril, Dichlorethan, Essigsäureethylester, Dioxan, Dimethylsulfoxid.

Die Reaktion ist nach einigen Stunden beendet, das Reaktionsprodukt kann durch Einengen der Mischung, Zugabe von Wasser und Extraktion mit einem unpolaren Lösungsmittel sowie Abdestillieren des Lösungsmittels unter vermindertem Druck, isoliert werden.

b) Darüber hinaus ist die Herstellung der neuen Verbindungen auch durch Umsetzung der Verbindungen II mit den entsprechenden Aminen $R^2-ONH_2$ durchführbar.

c) Weiterhin ist die Herstellung der neuen Derivate auch durch Alkylierung der Oxime mit Alkylierungsmitteln möglich

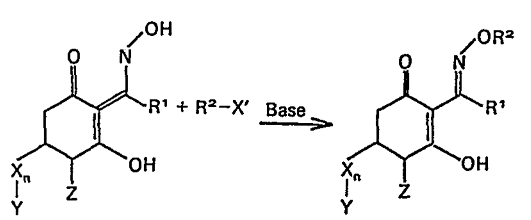


Das Verfahren a wird bevorzugt.

Die Verbindungen der Formel II können durch Acylierung der Cyclohexan-1,3-dione III, wie dies in „Tetrahedron Letters", *29*, 2491, beschrieben ist, erhalten werden. Die Verbindungen III können ebenfalls in tautomeren Formen vorliegen.

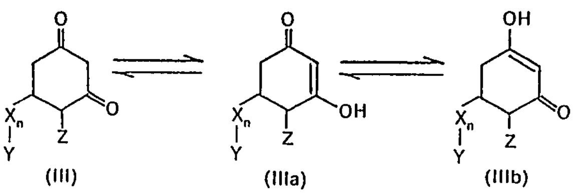

(III) (IIIa) (IIIb)

Verbindungen der Formel III sind aus Aldehyden $Y-X_n-CH=O$ nach literaturbekannten Methoden beispielsweise durch Aldolkondensation mit Ketonen und anschliessender Cyclisierung mit Malonsäureestern analog „Organic Synthesis Coll.", vol. II, S. 200, herstellbar. Auch durch Umsetzung des Aldehyds $Y-X_n-CH=O$ mit Malonsäure nach Knoevenagel-Döbner (s. „Org. Reaktions", Bd. *15*, S. 204), Verersterung der erhaltenen Säure sowie Cyclisierung mit Acetessigester, in analoger Weise wie dies z.B. in „Chem. Ber.", *96*, S. 2946, beschrieben wird, gelangt man zu den Zwischenprodukten der Formel III.

Die Salze der Verbindungen sind beispielsweise die Alkalisalze, insbesondere Natrium- oder Kaliumsalze.

Die Natrium- und Kaliumsalze der neuen Verbindungen können durch Behandeln dieser Verbindungen mit Natrium- oder Kaliumhydroxid in wässeriger Lösung oder in einem organischen Lösungsmittel wie Methanol, Ethanol, Aceton erhalten werden. Es können auch Alkalialkoholate als Basen eingesetzt werden.

Andere Metallsalze, z.B. die Mangan-, Kupfer-, Zink-, Eisen- oder Bariumsalze können aus dem Natriumsalz durch Reaktion mit dem entsprechenden Metallchlorid in wässeriger Lösung hergestellt werden. Die folgenden Beispiele erläutern die Herstellung der neuen Cyclohexandione (Gewichtsteile verhalten sich zu Volumenteilen wie Kilogramm zu Liter).

*Beispiel 1*

10,0 Gew.-Teile 2-Butyryl-4-methoxycarbonyl-5-(tetrahydropyran-4-ylmethyl)cyclohexan-1,3-dion wurden in 150 Vol.-Teilen Ethanol gelöst und mit 2,93 Gew.-Teilen Ethyloxiammoniumchlorid sowie 2,71 Gew.-Teilen wasserfreiem Natriumacetat versetzt. Nach 20stündigem Rühren bei 20°C wurde in Eiswasser gegeben und mit Methylenchlorid extrahiert. Nach dem Einengen der organischen Phase verblieben 10,5 Gew.-Teile 2-(1-Ethoxyaminobutyliden)-4-methoxycarbonyl-5-(tetrahydropyran-4-ylmethyl)cyclohexan-1,3-dion (Verbindung Nr. 1) als zähes Öl mit folgender Struktur

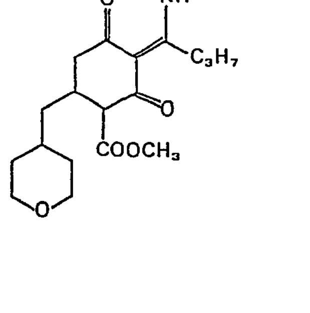

*Analyse für* $C_{20}H_{31}O_6N$ (M = 381)

| | | | |
|---|---|---|---|
| Berechnet: | C 63,0 | H 8,2 | N 3,7% |
| Gefunden: | C 63,3 | H 8,1 | N 3,7% |

*Beispiel 2*

10,0 Gew.-Teil 2-Butyryl-5-[2-(1,3-dioxan-2-yl)ethyl]cyclohexan-1,3-dion wurden in 150 Vol.-Teilen Ethanol gelöst und mit 3,72 Gew.-Teilen Allyloxiammoniumchlorid sowie 3,03 Gew.-Teilen wasserfreiem Natriumacetat versetzt und 20 h bei 20°C gerührt. Anschliessend wurde die Suspension in Eiswasser eingerührt und mit Methylenchlorid extrahiert. Nach Einengen der organischen Phase verblieben 11,5 Gew.-Teile 2-(1-Allyloxiaminobutyliden)-5-[2-(1,3-dioxan-2-yl)ethyl]cyclohexan-1,3-dion (Verbindung Nr. 2) als Feststoff mit folgender Struktur (Smp.: 50 bis 52°C)

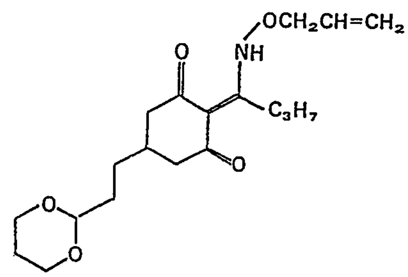

*Analyse für* $C_{19}H_{29}O_5N$ (M = 351)

| | | | |
|---|---|---|---|
| Berechnet: | C 64,9 | H 8,3 | N 4,0% |
| Gefunden: | C 65,1 | H 8,1 | N 3,7% |

*Beispiel 3*

12,0 Gew.-Teil 2-Butyryl-4-methoxycarbonyl-5-[2-(1,3-dithiolan-2-yl)ethyl]cyclohexan-1,3-dion wurden in 150 Vol.-Teilen Ethanol gelöst und mit 3,29 Gew.-Teilen Allyloxiammoniumchlorid sowie 3,28 g wasserfreiem Natriumacetat versetzt. Nach 20stündigem Rühren bei 20°C wurde auf Eiswasser gegeben und mit Methylenchlorid extrahiert. Nach Einengen der organischen Phase verblieben 13,1 Gew.-Teile 2-(1-Allyloxiaminobutyliden)-4-methoxycarbonyl-5-[2-(1,3-dithiolan-2-yl)ethyl]cyclohexan-1,3-dion (Verbindung Nr. 3) als zähes Öl mit nachstehender Struktur

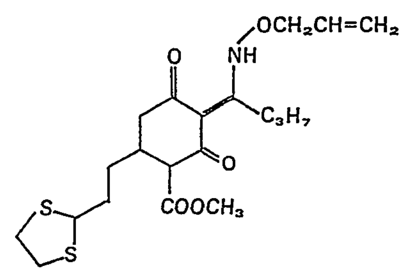

*Analyse für* $C_{20}H_{29}O_5NS_2$ (M = 427)

| | | | | |
|---|---|---|---|---|
| Berechnet: | C 56,2 | H 6,8 | N 2,3 | S 15,0% |
| Gefunden: | C 57,0 | H 6,7 | N 2,8 | S 14,7% |

Die folgenden Verbindungen wurden in entsprechender Weise erhalten.

| Verbindung Nr. | $R^1$ | $R^2$ | $X_n-Y$ | Z | Fp. oder Brechungsindex |
|---|---|---|---|---|---|
| 4 | Propyl | Allyl | Tetrahydropyran-4-ylmethyl | $COOCH_3$ | |
| 5 | Propyl | Ethyl | Tetrahydropyran-4-ylmethyl | H | |
| 6 | Propyl | Allyl | Tetrahydropyran-4-ylmethyl | H | |
| 7 | Propyl | Allyl | 2-(1,3-Dioxan-2-yl)ethyl | $COOCH_3$ | |
| 8 | Propyl | Ethyl | 2-(1,3-Dioxan-2-yl)ethyl | H | |
| 9 | Propyl | Allyl | 4-Methyltetrahydropyran-3-yl | $COOCH_3$ | |
| 10 | Propyl | Ethyl | 4-Methyltetrahydropyran-3-yl | H | $n_D^{22}$ = 1,5235 |
| 11 | Propyl | Allyl | 4-Methyltetrahydropyran-3-yl | H | $n_D^{22}$ = 1,5297 |
| 12 | Propyl | Ethyl | 1-(4-Methyl-1,3-dioxan-2-yl)-2-methylpropyl | $COOCH_3$ | |
| 13 | Propyl | Allyl | 1-(4-Methyl-1,3-dioxan-2-yl)-2-methylpropyl | $COOCH_3$ | |
| 14 | Propyl | Ethyl | 1-(4-Methyl-1,3-dioxan-2-yl)-2-methylpropyl | H | |
| 15 | Propyl | Ethyl | 1-Phenyl-2-(1,3-dioxolan-2-yl)ethyl | $COOCH_3$ | |
| 16 | Propyl | Allyl | 1-Phenyl-2-(1,3-dioxolan-2-yl)ethyl | $COOCH_3$ | |
| 17 | Propyl | Ethyl | [2H]-5,6-Dihydropyran-3-yl | $COOCH_3$ | |
| 18 | Propyl | Allyl | [2H]-5,6-Dihydropyran-3-yl | $COOCH_3$ | |
| 19 | Propyl | Ethyl | [2H]-5,6-Dihydropyran-3-yl | H | $n_D^{20}$ = 1,5339 |
| 20 | Propyl | Ethyl | [4H]-2,3-Dihydropyran-2-yl | $COOCH_3$ | $n_D^{26}$ = 1,5225 |
| 21 | Propyl | Allyl | [4H]-2,3-Dihydropyran-2-yl | $COOCH_3$ | $n_D^{27}$ = 1,5262 |
| 22 | Propyl | Ethyl | Tetrahydropyran-2-yl | $COOCH_3$ | $n_D^{24}$ = 1,5142 |
| 23 | Propyl | Allyl | Tetrahydropyran-2-yl | $COOCH_3$ | $n_D^{25}$ = 1,5204 |
| 24 | Propyl | Ethyl | Tetrahydropyran-2-yl | H | $n_D^{26}$ = 1,5136 |
| 25 | Propyl | Allyl | Tetrahydropyran-2-yl | H | $n_D^{27}$ = 1,5200 |
| 26 | Propyl | Ethyl | Tetrahydropyran-3-yl | H | $n_D^{24}$ = 1,5149 |
| 27 | Propyl | 3-Chlorallyl | Tetrahydropyran-4-ylmethyl | $COOCH_3$ | Fp. 75 bis 79°C |
| 28 | Propyl | 2-Chlorallyl | Tetrahydropyran-4-ylmethyl | $COOCH_3$ | Fp. 72 bis 75°C |
| 31 | Propyl | 3-Chlorallyl | Tetrahydropyran-4-ylmethyl | H | |
| 32 | Propyl | 2-Chlorallyl | Tetrahydropyran-4-ylmethyl | H | |
| 36 | Propyl | 2-Chlorallyl | 2-(1,3-Dioxan-2-yl)ethyl | H | Fp. 55 bis 58°C |
| 37 | Propyl | 3-Chlorallyl | 2-(1,3-Dioxan-2-yl)ethyl | H | $n_D^{22}$ = 1,5281 |
| 38 | Propyl | 2,3,3-Trichlorallyl | 2-(1,3-Dioxan-2-yl)ethyl | H | $n_D^{22}$ = 1,5401 |
| 41 | Propyl | 3-Chlorallyl | 4-Methyltetrahydropyran-3-yl | H | $n_D^{22}$ = 1,5389 |
| 44 | Propyl | Allyl | 1-Phenyl-2-(1,3-dioxolan-2-yl)ethyl | H | |
| 48 | Propyl | Ethyl | [4H]-2,5-Dimethyl-2,3-dihydropyran-2-yl | H | $n_D^{18}$ = 1,5259 |
| 49 | Propyl | Allyl | [4H]-2,5-Dimethyl-2,3-dihydropyran-2-yl | H | $n_D^{18}$ = 1,5301 |
| 61 | Propyl | Ethyl | [2H]-5,6-Dihydrothiopyran-3-yl | H | $n_D^{23}$ = 1,5620 |
| 62 | Propyl | Allyl | [2H]-5,6-Dihydrothiopyran-3-yl | H | $n_D^{23}$ = 1,5678 |
| 65 | Propyl | Ethyl | [2H]-2,6-Dimethyl-5,6-dihydrothiopyran-3-yl | H | $n_D^{23}$ = 1,5464 |
| 66 | Propyl | Allyl | [2H]-2,6-Dimethyl-5,6-dihydrothiopyran-3-yl | H | $n_D^{23}$ = 1,5510 |

Die folgenden Verbindungen können in entsprechender Weise erhalten werden.

| Verbindung Nr. | $R^1$ | $R^2$ | $X_n-Y$ | Z |
|---|---|---|---|---|
| 29 | Propyl | 2,3,3-Trichlorallyl | Tetrahydropyran-4-ylmethyl | $COOCH_3$ |
| 30 | Propyl | 2,3-Dibromallyl | Tetrahydropyran-4-ylmethyl | $COOCH_3$ |
| 33 | Propyl | 2,3-Dibromallyl | Tetrahydropyran-4-ylmethyl | H |
| 34 | Propyl | 3-Chlorallyl | 2-(1,3-Dioxan-2-yl)ethyl | $COOCH_3$ |
| 35 | Propyl | 2-Chlorallyl | 2-(1,3-Dioxan-2-yl)ethyl | $COOCH_3$ |
| 39 | Propyl | 2,3-Dichlorallyl | 2-(1,3-Dioxan-2-yl)ethyl | H |
| 40 | Propyl | 2,3-Dibromallyl | 2-(1,3-Dioxan-2-yl)ethyl | H |

| Verbindung Nr. | $R^1$ | $R^2$ | $X_n-Y$ | Z |
|---|---|---|---|---|
| 42 | Propyl | 3-Chlorallyl | 1-(4-Methyl-1,3-dioxan-2-yl)-2-methylpropyl | H |
| 43 | Propyl | 3-Chlorallyl | 1-Phenyl-2-(1,3-dioxolan-2-yl)ethyl | H |
| 45 | Propyl | Allyl | 2-(1,3-Dithiolan-2-yl)ethyl | H |
| 46 | Propyl | Ethyl | 2-(1,3-Dithiolan-2-yl)ethyl | H |
| 47 | Propyl | 3-Chlorallyl | 2-(1,3-Dithiolan-2-yl)ethyl | H |
| 50 | Propyl | 3-Chlorallyl | [4H]-2,5-Dimethyl-2,3-dihydropyran-2-yl | H |
| 51 | Propyl | Ethyl | 2,5-Dimethyltetrahydropyran-2-yl | H |
| 52 | Propyl | 3-Chlorallyl | 2,5-Dimethyltetrahydropyran-2-yl | H |
| 53 | Propyl | 3-Chlorallyl | [2H]-5,6-Dihydropyran-3-yl | H |
| 54 | Propyl | Ethyl | [4H]-2,3-Dihydropyran-2-yl | H |
| 55 | Propyl | Allyl | [4H]-2,3-Dihydropyran-2-yl | H |
| 56 | Propyl | 3-Chlorallyl | [4H]-2,3-Dihydropyran-2-yl | H |
| 57 | Propyl | 3-Chlorallyl | Tetrahydropyran-3-yl | H |
| 58 | Propyl | 3-Chlorallyl | Tetrahydropyran-2-yl | H |
| 59 | Propyl | 3-Chlorallyl | [2H]-2,6-Dimethyl-5,6-dihydrothiopyran-3-yl | H |
| 60 | Propyl | Ethyl | [2H]-5,6-Dihydrothiopyran-3-yl | $COOCH_3$ |
| 63 | Propyl | 3-Chlorallyl | [2H]-5,6-Dihydrothiopyran-3-yl | H |
| 64 | Propyl | Allyl | [2H]-2,6-Dimethyl-5,6-dihydrothiopyran-3-yl | $COOCH_3$ |

Die an diesen Verbindung festgestellten 1H-NMR-spektroskopischen Daten sind in folgender Tabelle aufgeführt. Die chemischen Verschiebungen wurden auf Tetramethylsilan als internen Standard bezogen und in δ-Werten (ppm) angegeben.

Als Lösungsmittel diente SCDI$_3$; Abkürzungen für die Signalstrukturen:

s Singulett
d Dublett
t Triplett
q Quartett
m Multiplett mit mehr als vier Linien

| Verbindung Nr. | 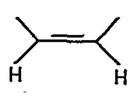 | Charakteristische Signale | |
|---|---|---|---|
| | | $O-CH_2$ | $COOCH_3$ |
| 1 | — | 4,09 (q) | 3,75 (s) |
| 2 | — | 4,51 (d) | — |
| 3 | — | 4,51 (d) | 3,77 (s) |
| 4 | — | 4,51 (d) | 3,78 (s) |
| 5 | — | 4,11 (q) | — |
| 6 | — | 4,52 (d) | — |
| 7 | — | 4,51 (d) | 3,76 (s) |
| 8 | — | 4,08 (q) | — |
| 9 | — | 4,50 (d) | 3,78 (s) |
| 10 | — | 4,08 (q) | — |
| 11 | — | 4,58 (d) | — |
| 12 | — | 4,09 (q) | 3,74 (s) |
| 13 | — | 4,54 (d) | 3,78 (s) |
| 14 | — | | |
| 15 | — | 4,06 (q) | 3,69 (s) |
| 16 | — | 4,51 (d) | 3,70 (s) |
| 17 | 5,75 (s) | | 3,78 (s) |
| 18 | 5,75 (s) | 4,50 (d) | 3,75 (s) |
| 19 | 5,60 (s) | 4,10 (q) | |
| 20 | 4,65 (m)<br>6,20 (m) | 4,10 (q) | 3,75 (s)* |

| Verbindung Nr. | 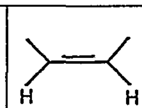 | Charakteristische Signale | |
|---|---|---|---|
| | | $O-CH_2$ | $COOCH_3$ |
| 21 | 4,70 (m)<br>6,30 (m) | 4,60 (d) | 3,70 (s) |
| 22 | — | 4,11 (q) | 3,75 (s)<br>3,80 (s) |
| 23 | — | 4,52 (d) | 3,75 (s)<br>3,80 (s) |
| 24 | — | 4,12 (q) | — |
| 25 | — | 4,51 (d) | — |
| 26 | — | 4,05 (q) | — |
| 31 | — | 4,50 (m) | — |
| 32 | — | 4,56 (s) | — |
| 44 | — | 4,50 | — |

* Die Aufspaltung der Estersignale wird durch Diasterometrie hervorgerufen.

Die Anwendung als Herbizid erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Streichen, Tränken, Vernebeln, Verstäuben, Verstreuen oder Giessen. Die Anwendungsformen richten sich ganz nach den Verwendungszwecken; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoran usw., stark polare Lösungsmittel, z.B. Dimethylformamid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Herbizide enthalten z.B. 5 bis 95% (Gew.-%), insbesondere 10 bis 80% Wirkstoff.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, äthoxyliertes Isooctylphenol-, Octylphenol-, Nonylphenol-, Alkylphenolpolyglykoläther, Tributylphenylpolyglykolether, Alkylarylpolyätheralkoholate, Isotridecylalkohol, Fettalkoholethylenoxid-Kondensate, ethoxyliertes Ricinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Silikate, Talkum, Kaolin, Kalk, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

*Beispiel a*

Man vermischt 90 Gew.-Teile der Verbindung 1 mit 10 Gew.-Teilen N-Methyl-α-pyrrolidon und erhält eine Lösung, die zur Anwendung in Form kleinster Tropfen geeignet ist.

*Beispiel b*

10 Gew.-Teile der Verbindung 2 werden in einer Mischung gelöst, die aus 90 Gew.-Teilen Xylol, 6 Gew.-Teilen des Anlagerungsproduktes von 8 bis 10 mol Äthylenoxid an 1 mol Ölsäure-N-monoäthanolamid, 2 Gew.-Teilen Calciumsalz der Dodecylbenzolsulfonsäure und 2 Gew.-Teilen des Anlagerungsproduktes von 40 mol Äthylenoxid an 1 mol Ricinusöl besteht.

*Beispiel c*

20 Gew.-Teile der Verbindung 3 werden in einer Mischung gelöst, die aus 60 Gew.-Teilen Cyclohexanon, 30 Gew.-Teilen Isobutanol, 5 Gew.-Teilen des Anlagerungsproduktes von 7 mol Äthylenoxid an 1 mol Isooctylphenol und 5 Gew.-Teilen des Anlagerungsproduktes von 40 mol Äthylenoxid an 1 mol Ricinusöl besteht.

*Beispiel d*

20 Gew.-Teile der Verbindung 1 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Äthylenoxid an 1 mol Ricinusöl besteht.

*Beispiel e*

80 Gew.-Teile des Wirkstoffs 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen.

*Beispiel f*

5 Gew.-Teile der Verbindung 1 werden mit 95 Gew.-Teilen feinteiligem Kaolin innig vermischt. Man erhält auf diese Weise ein Stäubemittel, das 5 Gew.-% des Wirkstoffs enthält.

*Beispiel g*

30 Gew.-% der Verbindung 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

*Beispiel h*

40 Gew.-Teile des Wirkstoffs 1 werden mit 10 Teilen Natriumsalz eines Phenolsulfonsäure/Harnstoff/Formaldehyd-Kondensats, 2 Teilen Kieselgel und 48 Teilen Wasser innig vermischt. Man erhält eine stabile wässerige Dispersion.

*Beispiel i*

20 Teile des Wirkstoffs 1 werden mit 12 Teilen Calciumsalz der Dodecylbenzolsulfonsäure, 8 Teilen Fettalkoholpolyglykoläther, 2 Teilen Natriumsalz eines Phenolsulfonsäure/Harnstoff/Formaldehyd-Kondensats und 68 Teilen eines paraffinischen Mineralöls innig vermischt. Man erhält eine stabile ölige Dispersion.

Die Wirkung der neuen Cyclohexan-1,3-dionderivate auf das Wachstum von Pflanzen aus der Gräserfamilie (Gramineen) und breitblättrigen Kulturpflanzen lässt sich durch Gewächshaus- und Freilandversuche zeigen. Dabei können auch Kulturpflanzen aus der Familie der Gramineen absterben oder stark geschädigt werden. Dies kann in der Praxis durchaus erwünscht sein, da auch Kulturpflanzen zu unerwünschten Pflanzen werden können, wenn sie aus im Boden zurückgebliebenem Samen in einer anderen Kultur aufwachsen, wie z.B. Ausfallgerste *(voluntary barley)* in Winterraps oder Soghum in Sojabohnenfeldern.

Als Kulturgefässe für die Versuche dienten Plastikblumentöpfe mit 300 $cm^3$ Inhalt und lehmigem Sand mit etwa 1,5% Humus als Substrat. Bei Soja wurde etwas Torf *(peat)* zugemischt, um ein besseres Wachstum zu gewährleisten. Die Samen der Testpflanzen wurden nach Arten getrennt flach eingesät.

Bei der Vorauflaufbehandlung wurden die Wirkstoffe auf die Erdoberfläche aufgebracht. Sie wurden hierzu in Wasser als Verteilungsmittel suspendiert oder emulgiert und mittels fein verteilender Düsen gespritzt. Bei dieser Applikationsmethode betrug die Aufwandmenge 3,0 kg Wirkstoff/ha. Nach dem Aufbringen der Mittel wurden die Gefässe leicht beregnet, um Keimung und Wachstum in Gang zu bringen. Danach deckte man die Gefässe mit durchsichtigen Plastikhauben ab, bis die Pflanzen angewachsen waren. Die Abdeckung bewirkte ein gleichmässiges Keimen der Testpflanzen, sofern dies nicht durch die Wirkstoffe beeinträchtigt wurde.

Zum Zwecke der Nachauflaufbehandlung zog man die Pflanzen je nach Wuchsform bis zu einer Wuchshöhe von 3 bis 15 cm an. Die Aufwandmengen für die Nachauflaufbehandlung variierte je nach Wirkstoff und Einsatzziel. Sie betrugen 0,125, 0,25, 0,5 und 1,0 kg Wirkstoff/ha.

Als Vergleichsbeispiel (DE-AS Nr. 2439104) dienten jeweils im Nachauflaufverfahren

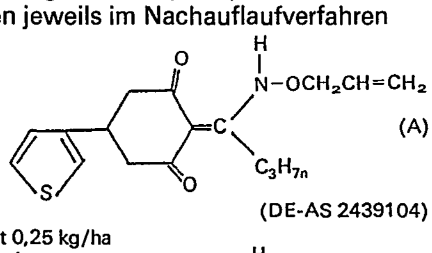

(A)

(DE-AS 2439104)

mit 0,25 kg/ha
sowie

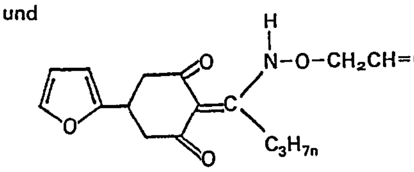

(B)

und



(C)

mit je 0,25 und 0,5 kg/ha.

Bei der Durchführung der Gewächshausversuche hielt man wärmeliebende Arten in wärmeren Bereichen (20 bis 35°C) und solche gemässigter Klimate bevorzugt bei 10 bis 20°C. Die Versuchsperiode erstreckte sich über 2 bis 4 Wochen. Während dieser Zeit wurden die Pflanzen gepflegt, und ihre Reaktion auf die einzelnen Behandlungen wurde ausgewertet. Bewertet wird nach einer Skala von 0 bis 100. Dabei bedeutet 0 keine Schädigung oder normaler Aufgang und 100 kein Aufgang bzw. völlige Zerstörung zumindest der oberirdischen Sprossteile.

Bei den ergänzend herangezogenen Feldversuchen wurden die Mittel auf Kleinparzellen ebenfalls in Wasser als Verteilungsmittel emulgiert oder suspendiert im Nachauflaufverfahren ausgebracht. Man benutzte hierzu eine auf einen Traktor montierte Parzellenspritze. Die Aufwandmengen betrugen 0,25 kg Wirkstoff/ha. Das Bekämpfungsziel war Ausfallgerste *(voluntary barley)* in jungem Winterraps.

Für die Darstellung der Ergebnisse wurden folgende Testpflanzen herangezogen.

*(Tabelle auf der nächsten Seite)*

Die Ergebnisse zeigen, dass die neuen Verbindungen bei Nachauflaufanwendung zur Bekämpfung von unerwünschten Pflanzen aus der Familie der Gräser (Gramineen) geeignet sind. Dabei kann es sich um typische Ungrasarten handeln, wie z.B. Flughafer *(Avena fatua)* oder um Kulturpflanzen aus der Familie der Gramineen, welche am falschen Standort wachsend zu unerwünschten Pflanzen werden (z.B. Mais in einem Sojabohnenfeld). Einzelne Verbindungen bekämpfen einerseits unerwünschte Gräser, andererseits weisen sie neben ihrer guten Selektivität für breitblättrige Kulturen gleichzeitig ein hohes Mass an Verträglichkeit für Weizen, der botanisch wiederum zur Gräserfamilie gehört, auf.

Die Prüfung der herbiziden Wirkung bei Nachauflaufanwendung von 0,25 kg Wirkstoff/ha der Verbindung Nr. 26 erbrachte gegen neun Beispielsgrasarten einen durchschnittlichen Bekämpfungswert von 81. Bei der selben Aufwandmenge und der gleichen Anwendungsmethode erreichte die Verbindung Nr. 24 einen Wert von 74.

Das bekannte Vergleichsmittel A hatte dagegen ebenfalls bei Nachauflaufanwendung von 0,25 kg Wirkstoff/ha gegen die selben Grasarten nur eine durchschnittliche Wirkung von 52%. Auch die beiden weiteren Vergleichsmittel B und C zeigten eine vergleichsweise nur schwache herbizide Aktivität.

Breitblättrige Kulturpflanzen, wie Baumwolle *(Gossypium hirsutum)*, Soja *(Glycine max.)*, Zukkerrüben *(Beta vulgaris)* und Raps *(Brassica napus)* blieben bei diesen Behandlungen völlig ohne Schädigung oder zeigten nur ganz unwesentliche Beeinträchtigungen des Wuchses. Daraus resultiert für die neuen Verbindungen ein hohes Mass an Selektivität für dikotyle Kulturen. Darüber hinaus bekämpften einzelne der neuen Verbindungen, wie z.B. die Nrn. 31 und 1, mit 0,25 kg Wirk-

| Botanischer Name | Deutscher Name | Englischer Name |
|---|---|---|
| *Alopecurus myosuroides* | Ackerfuchsschwanz | blackgrass |
| *Avena fatua* | Flughafer | wild oats |
| *Avena sativa* | Hafer | oats |
| *Beta vulgaris* | Zuckerrüben | sugarbeets |
| *Brassica napus* | Raps | rape seed |
| *Bromus tectorum* | Dach-Trespe | downy brome |
| *Echinochloa crus-galli* | Hühnerhirse | barnyardgrass |
| *Gossypium hirsutum* | Baumwolle | cotton |
| *Glycine max.* | Sojabohnen | soybeans |
| *Hordeum vulgare* | Gerste | barley |
| *Lolium multiflorum* | Ital. Raygras | annual ryegrass |
| *Rottboellia exaltata* | — | itchgrass |
| *Setaria spp.* | Borstenhirsearten | foxtail |
| *Sorghum bicolor* | Mohrenhirse | sorghum |
| *Sorghum halepense* | Sudangras | Johsongrass |
| *Tricticum aestivum* | Weizen | wheat |
| *Zea mays* | Mais | Indian corn |

stoff/ha unerwünschte Gräser, wie Ackerfuchsschwanz und Hirsen und verhielten sich dabei gleichzeitig selektiv für das Nutzgras Weizen.

Was die herbizide Aktivität betrifft, so konnte man in einer Reihe weiterer Beispiele die Wirkung der neuen Verbindungen gegen Pflanzenarten aus der Gräserfamilie nachweisen, z.B. die Nrn. 2, 10, 11, 19, 24 und 26.

In den beschriebenen Gewächshausversuchen erbrachten ferner bei Nachauflaufanwendung die Verbindung Nrn. 1, 4, 5, 8, 31, 32, 36 und 37 einen vergleichsweise guten Bekämpfungserfolg.

In Freilandversuchen wurde bei Nachauflaufanwendung von 0,25 kg Wirkstoff/ha der Verbindungen Nrn. 10, 11 und 26 Ausfallgerste in Raps selektiv bekämpft.

Neben den Nachauflaufwirkungen wurden auch positive Ergebnisse bei Vorauflaufanwendung der neuen Verbindungen im Gewächshaus erzielt. So wirkten bei 3,0 kg Wirkstoff/ha bei dieser Anwendungsmethode die Verbindungen Nrn. 2, 5, 8, 10, 14, 19, 26, 32, 36, 37, 48, 49, 54, 55 stark herbizid gegen die grasartigen Beispielspflanzen Hafer, Weidegras und Hühnerhirse. Ebenso hatten die Verbindungen Nrn. 1, 3 und 4 bei Vorauflaufanwendung von 3,0 kg Wirkstoff/ha im Gewächshaus eine beachtliche herbizide Aktivität gegen diese eben genannten Grasarten.

In Anbetracht der guten Verträglichkeit können die neuen Herbizide oder diese enthaltende Mittel noch in einer weiteren grossen Zahl von Kulturpflanzen zur Beseitigung unerwünschten Pflanzenwuchses eingesetzt werden. Die Aufwandmengen können dabei zwischen 0,025 und 15 kg/ha und mehr, vorzugsweise zwischen 0,1 und 5 kg/ha, schwanken.

Beispielsweise kommen folgende Kulturpflanzen in Betracht.

| Botanischer Name | Deutscher Name | Englischer Name |
|---|---|---|
| *Allium cepa* | Küchenzwiebel | onions |
| *Ananas comosus* | Ananas | pineapple |
| *Arachis hypogaea* | Erdnuss | peanuts (groundnuts) |
| *Asparagus officinalis* | Spargel | asparagus |
| *Beta vulgaris spp. altissima* | Zuckerrübe | sugarbeets |
| *Beta vulgaris spp. rapa* | Futterrübe | fodder beets |
| *Beta vulgaris spp. esculenta* | Rote Rübe | table beets, red beets |
| *Brassica napus var. napus* | Raps | rape seed |
| *Brassica napus var. napobrassica* | Kohlrübe | |
| *Brassica napus var. rapa* | Weisse Rübe | turnips |
| *Brassica rapa var. silvestris* | Rübsen | |
| *Camellia sinensis* | Teestrauch | tea plants |
| *Carthamus tinctorius* | Saflor – Färberdistel | safflower |
| *Carya illinoinensis* | Pekannussbaum | pecan trees |
| *Citrus limon* | Zitrone | lemon |
| *Citrus maxima* | Pampelmuse | grapefruits |
| *Citrus reticulata* | Mandarine | |
| *Citrus sinensis* | Apfelsine, Orange | orange trees |
| *Coffea arabica (Coffea canephora, Coffea liberica)* | Kaffee | coffee plants |
| *Cucumis melo* | Melone | melons |
| *Cucumis sativus* | Gurke | cucumber |
| *Cynodon dactylon* | Bermudagras | Bermudagrass in turfs and lawns |

| Botanischer Name | Deutscher Name | Englischer Name |
|---|---|---|
| *Daucus carota* | Möhre | carrots |
| *Elaesis guineensis* | Ölpalme | oil palms |
| *Fragaria vesca* | Erdbeere | strawberries |
| *Glyoine max.* | Sojabohne | soybeans |
| *Gossypium hirsutum (Gossypium arboreum Gossypium herbaceum Gossypium vitifolium)* | Baumwolle | cotton |
| *Helianthus annuus* | Sonnenblume | sunflowers |
| *Helianthus tuberosus* | Topinambur | |
| *Hevea brasiliensis* | Parakautschukbaum | rubber plants |
| *Humulus lupulus* | Hopfen | hop |
| *Ipomoea batatas* | Süsskartoffeln | sweet potato |
| *Juglans regia* | Walnussbaum | walnut trees |
| *Lactua sativa* | Kopfsalat | lettuce |
| *Lens culinaris* | Linse | lentils |
| *Linium usitatissimum* | Faserlein | flax |
| *Lycopersicon lycopersicum* | Tomate | tomato |
| *Malus spp.* | Apfel | apple trees |
| *Manihot esculenta* | Maniok | cassava |
| *Medicago sativa* | Luzerne | alfalfa (lucerne) |
| *Metha piperita* | Pfefferminze | peppermint |
| *Musa spp.* | Obst- und Mehlbanane | banana plants |
| *Nicotiana tabacum (N. rustica)* | Tabak | tobacco |
| *Olea europaea* | Ölbaum | olive trees |
| *Phaseolus lunatos* | Mondbohne | limabeans |
| *Phaseolus mungo* | Erdbohne | mungbeans |
| *Phaseolus vulgaris* | Buschbohne | snapbeans, green beans, dry beans |
| *Pennisetum glaucum* | Perl- oder Rohrkolbenhirse | |
| *Petroselinum crispum spp. tuberosum* | Wurzelpetersilie | parsley |
| *Picea abies* | Rotfichte | Norway spruce fire |
| *Abies alba* | Weisstanne | pine trees |
| *Pinus spp.* | Kiefer | cherry trees |
| *Pisum sativum* | Gartenerbse | plum trees |
| *Prunus avium* | Süsskirsche | cherry trees |
| *Prunus domestica* | Pflaume | plum trees |
| *Prunus dulcis* | Mandelbaum | almond trees |
| *Prunus persica* | Pfirsich | peach trees |
| *Pyrus communis* | Birne | pear trees |
| *Ribes sylvestre* | Rote Johannisbeere | red currants |
| *Ribes uva-crispa* | Stachelbeere | |
| *Ricinus communis* | Rizinus | |
| *Saccharum officinarum* | Zuckerrohr | sugar cane |
| *Sasamum indicum* | Sesam | Sesame |
| *Solanum tuberosum* | Kartoffel | Irish potatoes |
| *Sorghum bicolor (S. vulgare)* | Mohrenhirse (Unterblattspritzung) (post-directed) | sorghum |
| *Spinacia oleracea* | Spinat | spinach |
| *Theobroma cacao* | Kakaobaum | cacao plants |
| *Trifolium pratense* | Rotklee | red clover |
| *Triticum aestivum* | Weizen | wheat |
| *Vaccinium corymbosum* | Kulturheidelbeere | blueberry |
| *Vaccinium vitis-idaea* | Preisselbeere | cranberry |
| *Vicia faba* | Pferdebohne | tick beans |
| *Vigna sinensis (V. unguiculata)* | Kuhbohne | cow peas |
| *Vitis vinifera* | Weinrebe | grapes |
| *Zea mays* | Mais (Unterblattspritzung) (post-directed) | Indian corn, sweet corn maize |

Zur Verbreiterung des Wirkungsspektrums und zur Erzielung auch synergistischer Effekte können die neuen Cyclohexan-1,3-dionderivate mit bekannten Cyclohexan-1,3-dionderivaten und mit Zahlreichen Vertretern anderer herbizider oder wachstumsregulierender Wirkstoffgruppen gemischt und gemeinsam ausgebracht werden. Beispielsweise kommen als Mischungspartner Diazine, [4H]-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und andere in Frage.

Ausserdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nichtphytotoxische Öle und Ölkonzentrate zugesetzt werden.

**Patentansprüche** für die Vertragsstaaten BE, CH, DE, FR, GB, IT, LI, LU, NL, SE

1. Cyclohexandionderivat der allgemeinen Formel

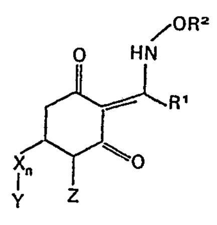

in der

$R^1$ Alkyl mit 1 bis 4 Kohlenstoffatomen,

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen,

X geradkettiger oder verzweigter Alkylenrest mit 1 bis 5 Kohlenstoffatomen, gegebenenfalls phenylsubstituiert,

n 0 oder 1,

Y einen nichtaromatischen Heterocyclus mit 5 oder 6 Atomen und keiner oder einer Doppelbindung im heterocyclischen Ring, enthaltend 1 oder 2 Heteroatome aus der Gruppe Schwefel oder Sauerstoff, wobei der Heterocyclus gegebenenfalls substituiert ist durch Alkyl in Form von Methyl, und

Z Wasserstoff oder Methoxycarbonyl

bedeutet, sowie die Salze dieser Verbindung.

2. Herbizid, enthaltend ein Cyclohexandionderivat gemäss Anspruch 1.

3. Herbizid, enthaltend einen festen oder flüssigen Trägerstoff und ein Cyclohexandionderivat gemäss Anspruch 1.

4. Verfahren zur Herstellung eines Herbizids, dadurch gekennzeichnet, dass man einen festen oder flüssigen Trägerstoff vermischt mit einem Cyclohexandionderivat gemäss Anspruch 1.

5. Verfahren zur Bekämpfung unerwünschten Pflanzenwuchses, dadurch gekennzeichnet, dass man den Boden oder die Pflanzen behandelt mit einem Cyclohexandionderivat gemäss Anspruch 1.

6. Verfahren zur Herstellung eines Cyclohexandionderivats gemäss Anspruch 1, dadurch gekennzeichnet, dass man eine Verbindung der allgemeinen Formel

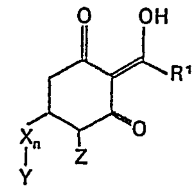

mit einer Ammoniumverbindung der Formel $R^2-O-NH_3^+A^-$, in denen $R^1$, $R^2$, X, Y, Z die in Anspruch 1 genannten Bedeutungen haben und $A^-$ ein Anion bedeutet, in einem inerten Lösungsmittel bei einem pH-Bereich von 2 bis 7 und bei Temperaturen zwischen 0 und 80°C umsetzt.

**Patentansprüche** für den Vertragsstaat: AT

1. Herbizid, enthaltend ein Cyclohexandionderivat der allgemeinen Formel

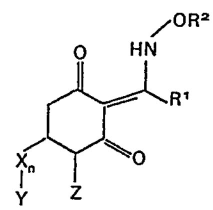

in der

$R^1$ Alkyl mit 1 bis 4 Kohlenstoffatomen,

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen,

X geradkettiger oder verzweigter Alkylenrest mit 1 bis 5 Kohlenstoffatomen, gegebenenfalls phenylsubstituiert,

n 0 oder 1,

Y einen nichtaromatischen Heterocyclus mit 5 oder 6 Atomen und keiner oder einer Doppelbindung im heterocyclischen Ring, enthaltend 1 oder 2 Heteroatome aus der Gruppe Schwefel oder Sauerstoff, wobei der Heterocyclus gegebenenfalls substituiert ist durch Alkyl in Form von Methyl, und

Z Wasserstoff oder Methoxycarbonyl

bedeutet, sowie die Salze dieser Verbindung.

2. Herbizid, enthaltend einen festen oder flüssigen Trägerstoff und ein Cyclohexandionderivat gemäss Anspruch 1.

3. Verfahren zur Herstellung eines Herbizids, dadurch gekennzeichnet, dass man einen festen

oder flüssigen Trägerstoff vermischt mit einem Cyclohexandionderivat gemäss Anspruch 1.

4. Verfahren zur Bekämpfung unerwünschten Pflanzenwuchses, dadurch gekennzeichnet, dass man den Boden oder die Pflanzen behandelt mit einem Cyclohexandionderivat gemäss Anspruch 1.

5. Verfahren zur Herstellung eines Cyclohexandionderivats gemäss Anspruch 1, dadurch gekennzeichnet, dass man eine Verbindung der allgemeinen Formel

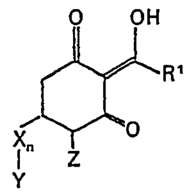

mit einer Ammoniumverbindung der Formel $R^2{-}O{-}NH_3^+A^-$, in denen $R^1$, $R^2$, X, Y, Z die in Anspruch 1 genannten Bedeutungen haben und $A^-$ ein Anion bedeutet, in einem inerten Lösungsmittel bei einem pH-Bereich von 2 bis 7 und bei Temperaturen zwischen 0 und 80° C umsetzt.

**Claims** for the contracting States BE, CH, DE, FR, GB, IT, LI, LU, NL, SE

1. A cyclohexandione derivative of the general formula

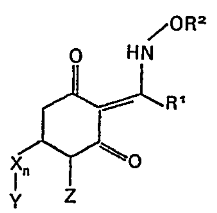

where

$R^1$ is alkyl of 1 to 4 carbon atoms,

$R^2$ is alkyl of 1 to 4 carbon atoms, alkenyl of 3 or 4 carbon atoms, alkynyl of 3 or 4 carbon atoms or haloalkenyl of 3 or 4 carbon atoms and 1 to 3 halogen atoms,

X is straight-chain or branched alkylene of 1 to 5 carbon atoms, which is unsubstituted or substituted by phenyl,

n is 0 or 1,

Y is a non-aromatic heterocyclic radical of 5 or 6 atoms and not more than one double bond in the heterocyclic ring, which contains 1 or 2 heteroatoms from the group comprising sulfur and oxygen and is unsubstituted or substituted by alkyl in the form of methyl, and

Z is hydrogen or methoxycarbonyl,

or a salt thereof.

2. A herbicide containing a cyclohexanedione derivative as claimed in Claim 1.

3. A herbicide containing a solid or liquid carrier and a cyclohexanedione derivative as claimed in Claim 1.

4. A process for producing a herbicide, wherein a solid or liquid carrier is mixed with a cyclohexanedione derivative as claimed in Claim 1.

5. A process for combating unwanted plant growth, wherein the soil or the plants are treated with a cyclohexanedione derivative as claimed in Claim 1.

6. A process for preparing a cyclohexanedione derivative as claimed in Claim 1, wherein a compound of the general formula

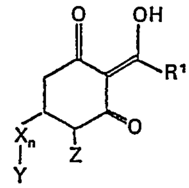

is reacted with an ammonium compound of the formula $R^2{-}O{-}NH_3^+A^-$ where $R^1$, $R^2$, X, Y and Z have the meanings given in Claim 1 and $A^-$ is an anion, in an inert solvent, at a pH of from 2 to 7, and at from 0 to 80°C.

**Claims** for the contracting State: AT

1. A herbicide containing a cyclohexanedione derivative of the general formula

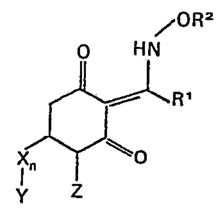

where

$R^1$ is alkyl of 1 to 4 carbon atoms,

$R^2$ is alkyl of 1 to 4 carbon atoms, alkenyl of 3 or 4 carbon atoms, alkynyl of 3 or 4 carbon atoms or haloalkenyl of 3 or 4 carbon atoms and 1 to 3 halogen atoms,

X is straight-chain or branched alkylene of 1 to 5 carbon atoms, which is unsubstituted or substituted by phenyl,

n is 0 or 1,

Y is a non-aromatic heterocyclic radical of 5 or 6 atoms and not more than one double bond in the heterocyclic ring, which contains 1 or 2 heteroatoms from the group comprising sulfur and oxygen and is unsubstituted or substituted by alkyl in the form of methyl, and

Z is hydrogen or methoxycarbonyl,

or a salt thereof.

2. A herbicide containing a solid or liquid carrier and a cyclohexanedione derivative as defined in Claim 1.

3. A process for producing a herbicide, wherein a solid or liquid carrier is mixed with a cyclohexanedione derivative as defined in Claim 1.

4. A process for combating unwanted plant growth, wherein the soil or the plants are treated with a cyclohexanedione derivative as defined in Claim 1.

5. A process for preparing a cyclohexanedione derivative as defined in Claim 1, wherein a compound of the general formula

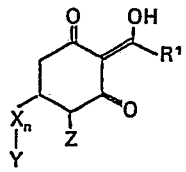

is reacted with an ammonium compound of the formula $R^2-O-NH_3^+A^-$ where $R^1$, $R^2$, X, Y and Z have the meanings given in Claim 1 and $A^-$ is an anion, in an inert solvent, at a pH of from 2 to 7, and at from 0 to 80°C.

**Revendications** pour les Etats contractants BE, CH, DE, FR, GB, IT, LI, LU, NL, SE

1. Dérivé de cyclohexanedione de formule générale:

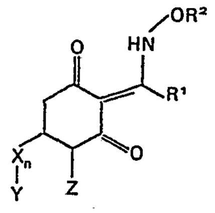

dans laquelle

$R^1$ représente alkyle à 1 à 4 atomes de carbone,

$R^2$ alkyle à 1 à 4 atomes de carbone, alcényle à 3 à 4 atomes de carbone, alcinyle à 3 à 4 atomes de carbone ou halogénalcényle à 3 ou 4 atomes de carbone et 1 à 3 atomes d'halogène,

X un reste alkylène à 1 à 5 atomes de carbone, à chaîne droite ou ramifiée, éventuellement substitué par phényle,

n 0 ou 1,

Y un hétérocycle non aromatique à 5 ou 6 atomes et n'ayant pas de double liaison ou ayant une seule liaison dans le noyau hétérocyclique, contenant 1 ou 2 hétéroatomes du groupe soufre ou oxygène, l'hétérocycle étant éventuellement substitué par alkyle sous forme de méthyle, et

Z hydrogène ou méthoxycarbonyle,

ainsi que les sels de ce composé.

2. Herbicide contenant un dérivé de cyclohexanedione selon la revendication 1.

3. Herbicide contenant un support solide ou liquide et un dérivé de cyclohexanedione selon la revendication 1.

4. Procédé de préparation d'un herbicide, caractérisé par le fait qu'on mélange un support solide ou liquide avec un dérivé de cyclohexanedione selon la revendication 1.

5. Procédé de lutte contre la croissance indésirable de plantes, caractérisé par le fait que l'on traite le sol ou les plantes avec un dérivé de cyclohexanedione selon la revendication 1.

6. Procédé de préparation d'un dérivé de cyclohexanedione selon la revendication 1, caractérisé par le fait que l'on fait réagir, dans un solvant inerte, dans une zone de pH de 2 à 7 et à des températures comprises entre 0 et 80°C, un composé de formule générale

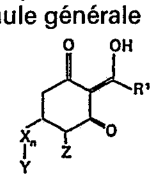

avec un dérivé d'ammonium de formule $R^2-O-NH_3^+A^-$, où $R^1$, $R^2$, X, Y, Z ont les significations données dans la revendication 1 et $A^-$ représente un anion.

**Revendications** pour l'Etat contractant: AT

1. Herbicide contenant un dérivé de cyclohexanedione de formule générale

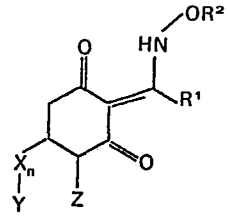

dans laquelle

$R^1$ représente alkyle à 1 à 4 atomes de carbone,

$R^2$ alkyle à 1 à 4 atomes de carbone, alcényle à 3 à 4 atomes de carbone, alcinyle à 3 à 4 atomes de carbone ou halogénalcényle à 3 ou 4 atomes de carbone et 1 à 3 atomes d'halogène,

X un reste alkylène à 1 à 5 atomes de carbone, à chaîne droite ou ramifiée, éventuellement substitué par phényle,

n 0 ou 1,

Y un hétérocycle non aromatique à 5 ou 6 atomes et n'ayant pas de double liaison ou ayant une seule liaison dans le noyau hétérocyclique, contenant 1 ou 2 hétéroatomes du groupe soufre ou oxygène, l'hétérocycle étant éventuellement substitué par alkyle sous forme de méthyle, et

Z hydrogène ou méthoxycarbonyle,

ainsi que les sels de ce composé.

2. Herbicide contenant un support solide ou liquide et un dérivé de cyclohexanedione selon la revendication 1.

3. Procédé de préparation d'un herbicide, caractérisé par le fait qu'on mélange un support solide ou liquide avec un dérivé de cyclohexanedione selon la revendication 1.

4. Procédé de lutte contre la croissance indésirable de plantes, caractérisé par le fait que l'on traite le sol ou les plantes avec un dérivé de cyclohexanedione selon la revendication 1.

5. Procédé de préparation d'un dérivé de cyclohexanedione selon la revendication 1, caractérisé par le fait que l'on fait réagir, dans un solvant inerte, dans une zone de pH de 2 à 7 et à des températures comprises entre 0 et 80°C, un composé de formule générale

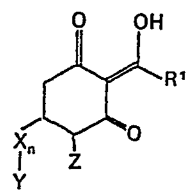

avec un dérivé d'ammonium de formule $R^2-O-NH_3^+A^-$, où $R^1$, $R^2$, X, Y, Z ont les significations données dans la revendication 1 et $A^-$ représente un anion.